Europäisches Patentamt

European Patent Office

Office européen des brevets

(11) Veröffentlichungsnummer: **0 243 843**

**A1**

(12) **EUROPÄISCHE PATENTANMELDUNG**

(21) Anmeldenummer: **87105797.2**

(22) Anmeldetag: **18.04.87**

(51) Int. Cl.4: **A61F 5/48**

(30) Priorität: **02.05.86 DE 3614988**

(43) Veröffentlichungstag der Anmeldung:
**04.11.87 Patentblatt 87/45**

(84) Benannte Vertragsstaaten:
**AT BE CH DE ES FR GB GR IT LI LU NL SE**

(71) Anmelder: **Wilde Membran Impuls Technik GmbH**
**Pregelstrasse 2**
**D-5828 Ennepetal(DE)**

(72) Erfinder: **Wilde, Horst D.**
**Pregelstrasse 2a**
**D-5828 Ennepetal(DE)**
Erfinder: **Bonack, Armin**
**Junkerstrasse 10**
**D-5650 Solingen(DE)**

(74) Vertreter: **Patentanwaltsbüro Cohausz & Florack**
**Postfach 14 01 47**
**D-4000 Düsseldorf 1(DE)**

(54) Feuchtigkeitsfühler für ein Gerät zur Enuresistherapie.

(57) Die Erfindung bezieht sich auf einen Feuchtigkeitsfühler für ein Gerät zur Enuresistherapie, das bei festgestellter Feuchtigkeit mittels eines elektronischen Signalgebers ein Signal gibt. Der aus einem Träger für die Elektroden und aus einem mit dem Träger verbundenen Band für die Anschlußleitungen bestehende Feuchtigkeitsfühler weist ein Faservlies (1,2) aus thermisch verschweißten Hochdruckpolyäthylen-Fasern mit darauf nach dem Siebdruckverfahren gedruckten Leitungszügen (3,4,3a bis 3b,4,4a bis 4c) sowie eine dünne Vliesstofflage (7) auf, die im Bereich der die Elektroden (3a bis 4b,4a bis 4c) bildenden Enden der Leitungen aufkaschiert ist. Die Leitungszüge (3,4) im übrigen Bereich sind durch einen Streifen (8) aus dem gleichen papierartigen Faservlies abgedeckt.

Ein solcher Feuchtigkeitsfühler stellt einen Wegwerfartikel dar. Er zeichnet sich durch seine geringe Dicke, hohe Flexibilität und Hautfreundlichkeit aus.

Fig.1

## Feuchtigkeitsfühler für ein Gerät zur Enuresistherapie

Die Erfindung bezieht sich auf einen Feuchtigkeitsfühler für ein einen Signalgeber aufweisendes Gerät zur Enuresistherapie, bestehend aus einem flachen porösen Träger für zwei auf gegenseitigen Abstand gehaltene Elektroden und aus einem mit dem Träger verbundenen Flachband, in dem mit den Elektroden verbundene, gegeneinander isolierte Leitungen fixiert sind und das am freien Ende mit den Leitungen verbundene Anschlußkontakte, insbesondere in Form von Druckknöpfen trägt, an die der Signalgeber mit entsprechenden Anschlußkontakten anschließbar ist.

Bei einem bekannten, seit Jahren erfolgreich bei der Enuresisbehandlung eingesetzten Gerät ist der elektronisch gesteuerte Signalgeber in einem kleinen Gehäuse untergebracht, das mit einem Trägergurt auf der Schulter nah dem Ohr des Patienten befestigt wird. Der Gurt ist als Flachband ausgebildet und trägt die gegeneinander isolierten Anschlußleitungen des elektronisch gesteuerten Signalgebers. Diese Anschlußleitungen sind mittels Druckknopfkontakten mit den Anschlußleitungen des wechselbaren Feuchtigkeitsfühlers verbunden. Der Feuchtigkeitsfühler besteht aus einem weichen aber strapazierfähigen Stoffteil, in dem die Elektroden eingewebt sind. Die mit den Elektroden verbundenen Leitungen sind in dem als Gurt ausgebildeten Flachband eingebettet, das an dem Stoffteil angenäht ist. An dem Stoffteil sind weitere Befestigungsmittel in Form von Druckknöpfen angebracht, mit denen der die Elektroden umfassende Stoffteil in der Unterhose des Patienten unmittelbar vor der Harnröhrenöffnung fixiert werden kann. Für die Behandlung eines Patienten müssen mehrere solcher Feuchtigkeitsfühler zur Verfügung stehen, denn wenn der Feuchtigkeitsfühler durch Harn feucht geworden ist und zur Auslösung eines Signals geführt hat, muß er gegen einen trockenen ausgewechselt werden können.

Von Nachteil ist bei solchen Geräten zur Enuresistherapie der hohe Herstellungsaufwand der Feuchtigkeitsfühler. Aus diesem Grunde werden die Feuchtigkeitsfühler nach jeder Benutzung gewaschen und getrocknet, damit sie immer wieder benutzt werden können. Aus dem Gesichtspunkt der Hygiene ist diese Methode bedenklich.

Aufgabe der Erfindung ist es, einen Feuchtigkeitsfühler der eingangs genannten Art als Wegwerfartikel zu schaffen.

Diese Aufgabe wird erfindungsgemäß durch folgende Merkmale gelöst:

(a) Der flache poröse Träger besteht aus einem papierartigen Faservlies aus thermisch verschweißten Hochdruckpolyäthylen-Fasern und einem einseitig aufkaschierten Vliesstoff.

(b) Das Flachband besteht aus dem gleichen papierartigen Faservlies wie der Träger und ist mit diesem einstückig verbunden.

(c) Die Elektrode im Träger und die damit verbundenen Leitungen im Flachband sind als auf dem papierartigen Faservlies nach dem Siebdruckverfahren gedruckte Leitungszüge ausgebildet.

(d) Die auf dem papierartigen Faservlies des Flachbandes aufgedruckten Leitungszüge sind durch einen aufkaschierten Papier-oder Folienstreifen, insbesondere aus dem gleichen papierartigen Faservlies wie der Träger, oder Isolierlack, abgedeckt.

Der erfindungsgemäße Feuchtigkeitsfühler ist aufgrund seines Aufbaus und der ausgewählten Materialien mit geringem Aufwand herzustellen, so daß der frühere Aufwand des Waschens und Trocknens für die mehrmalige Benutzung nicht länger lohnt. Als Wegwerfartikel genügt er höchsten Hygieneansprüchen. Da für den aufkaschierten Vliesstoff die Artikel der Intimhygiene Verwendung finden, ist der erfindungsgemäße Feuchtigkeitsfühler auch angenehmer an der Haut zu tragen als die herkömmlichen Feuchtigkeitsfühler bei denen selbst weicher Stoff vergleichsweise hart ist. Schließlich ist das die Leitungszüge und den Vliesstoff tragende papierartige Faservlies im Vergleich zu dem Stoffteil und Leinengurt des bekannten Feuchtigkeitsfühlers um ein vielfaches dünner und flexibler. Es trägt also weniger auf und stört weniger beim Tragen. Das papierartige Faservlies ist bei seiner hohen Flexibilität praktisch dehnfrei. Das bringt einen doppelten Vorteil: Das Faservlies entlastet den Vliesstoff von Zugbeanspruchungen, so daß dieser sehr dünn sein kann und damit die Feuchtigkeit schnell zu den Elektroden durchdringen kann. Ferner lassen sich darauf die Leitungszüge aufdrucken. Bewährt haben sich Leitungszüge aus aufgedruckter Graphitpaste.

Beim bekannten Feuchtigkeitsfühler hängt die Zeit zwischen dem Ansprechen und dem Anfang des Eindringens von Harn in den Stoffteil von der Lage des Ortes, wo der Harn in den Stoffteil eintritt, zur Lage der Elektroden und von der materialbedingten Ausbreitungsgeschwindigkeit der Feuchtigkeit im Stoffteil ab. Das bedeutet, daß es zu nicht unerheblichen Verzögerungen zwischen dem Beginn des Harnlassens und der Signalauslösung kommen kann. Diese Verzögerungszeit kann beim erfindungsgemäßen Feuchtigkeitsfühler auf einfache Art und Weise nach einer weiteren Ausgestaltung der Erfindung erheblich verkürzt werden, und zwar dadurch, daß die beiden Elektroden vielfach verzweigt sind, wobei die Zweige ineinander greifen und ein über die Oberfläche des papierartigen Faservlieses verteiltes Netz bilden. Bei dieser Ausgestaltung kommt es nicht mehr auf die räumliche Zuordnung zwischen der Harnröhrenöffnung und einem bestimmten Ort des flächigen Feuchtigkeitsfühlers an, denn durch die besondere Verteilung der Leiterzüge ist die gesamt Fläche des Feuchtigkeitsfühlers auf Feuchtigkeit sensibel. Deshalb spielt auch die Ausbreitungsgeschwindigkeit im Faservlies keine entscheidende Rolle mehr.

Um den Feuchtigkeitsfühler möglichst einfach zu positionieren, kann die freie Seite des papierartigen Faservlieses des Trägers mindestens eine Klebemarke als lösbares Befestigungselement tragen. Diese Art von Befestigung ist an sich von den für Frauen bestimmten Artikel der Intimhygiene bekannt.

Im folgenden wird die Erfindung anhand einer ein Ausführungsbeispiel darstellenden Zeichnung näher erläutert. Im einzelnen zeigen:

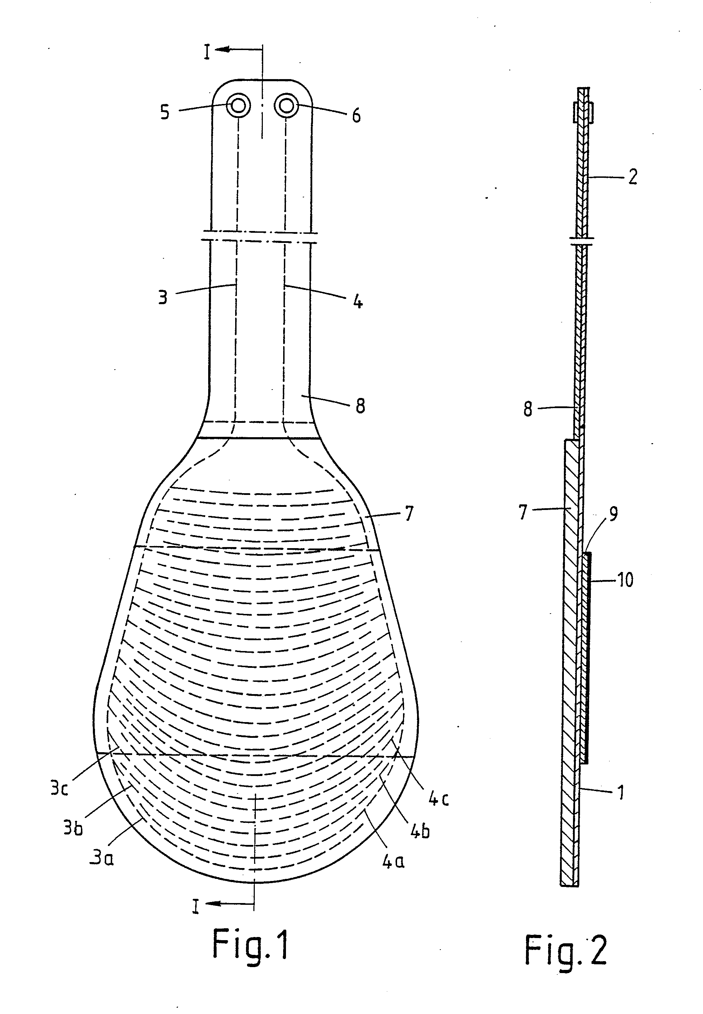

Fig. 1 einen Feuchtigkeitsfühler in Aufsicht und

Fig. 2 den Feuchtigkeitsfühler gemäß Fig. 1 im Querschnitt nach der Linie I - I der Fig. 1

Ein Zuschnitt aus einem papierartigen Faservlies aus thermisch verschweißten Hochdruckpolyäthylen-Fasern - ein unter dem Markennamen Tyvek bekanntes Produkt der Firma Du Pont - besteht aus einem großflächigen Teil 1 und einem damit einstückig verbundenen bandförmigen Teil 2. Auf diesem sehr flexiblen aber nicht dehnfähigen Faservlies sind einseitig nach dem Siebdruckverfahren aus Graphitpaste Leitungszüge aufgedruckt. Die Leitungszüge auf dem größeren Teil 1 bestehen aus zwei Gruppen von verzahnt ineinandergreifenden Zweigen 3a,3b,3c,4a,4b,4c, die mit den Leitungen 3,4 auf dem bandartigen Teil 2 verbunden sind. Die freien Enden der Leitungen 3,4 sind elektrisch mit metallenen Druckknöpfen 5,6 verbunden, die im bandartigen Teil 2 eingestanzt sind und mit entsprechenden Druckknöpfen in einem Gurt verbindbar sind, der die zu dem Signalgeber führenden Leitungen trägt.

Die Leitungszüge 3a bis 3c,4a bis 4c sind durch eine dünne, aufkaschierte Vliesstofflage 7 abgedeckt. Diesem Vliesstoff kommt nur die Aufgabe zu, hautfreundlich zu sein und die Feuchtigkeit aufzunehmen. Er braucht keine Zugfestigkeit zu haben, weil die Zugfestigkeit durch das papierartige Faservlies aufgebracht wird.

Im Bereich des bandartigen Teils 2 sind die Leitungen 3,4 durch einen Papier-oder Folienstreifen 8, insbesondere aus dem gleichen papierartigen Faservlies, abgedeckt und damit gegeneinander isoliert.

Auf der freien Rückseite trägt der größere Teil 1 einen Klettverschluß oder eine selbstklebende Klebemarke 9, die durch ein abziehbares Schutzpapier 10 abgedeckt ist. Mittels dieser Klebemarke 9 läßt sich der Feuchtigkeitsfühler an der Innenseite der Unterhose unmittelbar vor der Harnröhrenöffnung festmachen.

## Ansprüche

1. Feuchtigkeitsfühler für ein einen Signalgeber aufweisendes Gerät zur Enuresistherapie, bestehend aus einem flachen porösen Träger für zwei auf gegenseitigen Abstand gehaltene Elektroden und aus einem mit dem Träger verbundenen Flachband, in dem mit den Elektroden verbundene, gegeneinander isolierte Leitungen fixiert sind und das am freien Ende mit den Leitungen verbundene Anschlußkontakte, insbesondere in Form von Druckknöpfen trägt, an die der Signalgeber mit entsprechenden Anschlußkontakten anschließbar ist,
**gekennzeichnet durch** folgende Merkmale:

(a) Der flache poröse Träger besteht aus einem papierartigen Faservlies (1) aus thermisch verschweißten Hochdruckpolyäthylen-Fasern und einseitig aufkaschiertem Vliesstoff (7).

(b) Das Flachband besteht aus dem gleichen papierartigen Faservlies (2) wie der Träger und ist mit diesem einstückig verbunden.

(c) Die Elektroden (3a,3b,3c,4a,4b,4c) im Träger und die damit verbundenen Leitungen (3,4) im Flachband sind als auf dem papierartigen Faservlies (1,2) nach dem Siebdruckverfahren gedruckte Leitungszüge ausgebildet.

(d) Die auf dem papierartigen Faservlies (2) des Flachbandes aufgedruckten Leitungszüge (3,4) sind durch einen aufkaschierten Papier-oder Folienstreifen (8), insbesondere aus dem gleichen papierartigen Faservlies wie der Träger, oder Isolierlack abgedeckt.

2. Feuchtigkeitsfühler nach Anspruch 1,
**dadurch gekennzeichnet,** daß die Leitungszüge (3,3a bis 3c,4,4a bis 4c) aus einer aufgedruckten Graphitpaste bestehen.

3. Feuchtigkeitsfühler nach Anspruch 1 oder 2,
**dadurch gekennzeichnet,** daß die beiden Elektroden vielfach verzweigt sind, wobei die Zweige (3a bis 3c,4a bis 4c) ineinandergreifen und ein über die Oberfläche des Faservlieses (1) verteiltes Netz bilden.

4. Feuchtigkeitsfühler nach einem der Ansprüche 1 bis 3,
**dadurch gekennzeichnet,** daß die freie Seite des papierartigen Faservlieses (1) des Trägers mindestens eine Klebemarke (9) als lösbares Befestigungselement an einer Unterhose trägt.

I

5    6

3    4

8

7

3c    4c
3b    4b
3a    4a

I

**Fig.1**

2

8

7    9
10

1

**Fig.2**

### Europäisches Patentamt

## EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-2 552 553  (MEDTRON ELECTRONICS PTY. LTD.) * Ansprüche 1-3; Seite 6,  Zeilen 3-4 * | 1 | A 61 F    5/48 |
| A | | 2 | |
| | --- | | |
| Y | FR-A-2 441 383  (LE LEVIER) *  Seite  2, Zeilen 2-21; Figuren 1, 2 * | 1 | |
| | --- | | |
| Y | US-A-3 809 078  (MOZES) * Figur 1 * | 1 | |
| | --- | | |
| A | US-A-3 696 357  (KILGORE) * Figur 1; Spalte 3, Zeilen 26-38 * | 1,4 | RECHERCHIERTE SACHGEBIETE (Int. Cl.4) |
| | --- | | A 61 F    5/00 |
| A | CH-A-  532 395  (DOLDER) * Figur * | 1,3 | |
| | --- | | |
| A | FR-A-2 522 960  (HUET) * Anspruch 1; Figur 1 * | 1-3 | |
| | ----- | | |

Der vorliegende Recherchenbericht wurde für alle Patentansprüche erstellt.

| Recherchenort | Abschlußdatum der Recherche | Prüfer |
|---|---|---|
| BERLIN | 10-07-1987 | KANAL P K |